# EUROPEAN PATENT SPECIFICATION

(11) **EP 0 922 451 B1**
(45) Date of publication and mention of the grant of the patent: **25.06.2003**
(21) Application number: 98122633.5
(22) Date of filing: 27.11.1998
(51) Int. Cl.: A61K 9/52, A01N 1/02, A61K 35/52

(54) **Microcapsules containing seminal material for artificial insemination in pigs**
Spermamaterial enthaltende Mikrokapseln für künstliche Besamung von Schweinen
Microcapsules contenant du matériau de sperme pour l' insémination artificielle chez le cochon

(30) Priority: 28.11.1997 IT MI972652
(43) Date of publication of application: 16.06.1999
(73) Proprietor: Università di Pavia, 27100 Pavia (IT); UNIVERSITA' DEGLI STUDI DI MILANO, I-20122 Milan (IT)
(72) Inventor: Conte, Ubaldo, 21052 Busto Arsizio (IT); Torre, Maria Luisa Marina, 27100 Pavia (IT); Maggi, Lauretta, 27100 Pavia (IT); Giunchedi, Paolo, 27051 Cava Manara (IT); Vigo, Daniele, 27046 Santa Giuletta (IT); Maffeo, Giovanni, 20075 Lodi (IT); Russo, Vincenzo, 20135 Milan (IT)
(74) Representative: Gervasi, Gemma, Dr.

(56) References cited:
- US-A- 4 840 891
- R.L. NEBEL ET AL.: "MICROENCAPSULATION OF BOVINE SPERMATOZOA FOR USE IN ARTIFICIAL INSEMINATION: A REVIEW." REPRODUCTION, FERTILITY AND DEVELOPMENT, vol. 5, no. 6, 1993, pages 701-712, XP000891485 MELBOURNE, AU
- PATENT ABSTRACTS OF JAPAN vol. 012, no. 047 (C-475), 12 February 1988 (1988-02-12) & JP 62 195284 A (KIBUN KK), 28 August 1987 (1987-08-28)
- PATENT ABSTRACTS OF JAPAN vol. 013, no. 372 (C-627), 17 August 1989 (1989-08-17) & JP 01 128787 A (KOMATSU LTD), 22 May 1989 (1989-05-22)

## Description

### Scope of invention

The present invention regards microcapsules that may be used for artificial insemination in pigs, containing pig seminal material, and the corresponding process of preparation.

These capsules make it possible to obtain a prolonged and controlled release of seminal material and in addition a protection of the material from degradation, thus enabling a simplification in the procedure of artificial insemination and an increase in pig fertility.

### State of the art

In the last few decades, artificial insemination (A.I.) in cattle has witnessed a considerable diffusion among stock breeders, enabling insemination to be carried out on the basis of precise physiological data which make it possible to intervene with considerable precision and high probability of success.

This technique involves almost exclusively the use of fresh and/or refrigerated semen, the preparation of which is carried out at farm A.I. centres, also with the use of seminal material coming from external supplies.

For improving the likelihood of success of artificial insemination, numerous techniques have been developed, above all in the field of cattle for milk production.

In some domestic species, the technique of freezing fresh seminal material has led to a standardization of the methods of preservation and good likelihood of success of artificial insemination (e.g., cattle).

Among the methods used to improve the possibility of fertilization in cattle, it may be recalled that Nebel, as many as 15 years ago, attempted a series of modifications of artificial insemination in cattle using micro-encapsulated material instead of fresh seminal material (see R.L. Nebel, et al., J. Anim. Sci., 60, 1631 (1985); R.L. Nebel, *et al*., Reprod. Fertil. Dev., 5, 701 (1993)) and adopting a technique that involved the use of alginates and polyamines (poly I-lysine, protamine sulphate, polyvinyl amine) to obtain microcapsules consisting of semipermeable membranes. This procedure comprised three steps: 1) formation of a matrix of calcium alginate containing the seminal material; 2) formation of the semipermeable membrane by means of surface reticulation of the matrix with polyamines; 3) dissolution of the matrix nucleus of calcium alginate by substitution of calcium with sodium. With this procedure, then, the seminal material was initially immobilized in the calcium alginate matrix, and subsequently suspended in sodium alginate.

This technique of protection of bovine seminal material was quite successful in so far as this material is particularly stable and sustains micro-encapsulation treatments without excessive power loss.

On the other hand, artificial insemination (A.I.) is particularly problematical in pigs in so far as the technique of freezing, which has led to a good standardization of the methods of preservation in other domestic species, is practically inapplicable for pig seminal material, in that there exist considerable differences of efficiency of preservation using the freezing process.

The results acquired experimentally with frozen semen have made it possible to reveal excessive variability of parameters, such as fertility and prolificacy. In particular, the reduction of percentages of prolificacy and fertility has been identified in a slowing-down and/or failure of the embryo to grow during the first 4-5 days of life (see F. Cairoli *et al.,* Selezion Veterinaria, 32 (1 bis), 297 (1991)).

In addition, pigs present a duration of oestrus that ranges from 40 to 48 hours, with a follicular and ovulatory dynamics influenced by several factors, both of endogenous and exogenous type. For this reason, technicians tend to carry out a double A.I. intervention in order to guarantee the presence, at the tubal level, of a high concentration of spermatozoa with high fertilizing capacity. Obviously, the double-intervention technique represents a considerable inconvenience both on account of the need for resorting to specialized operators and on account of the high consumption of seminal material.

The reduction in the fertilizing capacity of the spermatozoa is very probably one of the causes that limit prolificacy. A high incidence of the loss of fertilizing capacity is found also as a result of the problematical aspects of the processes of preservation of seminal material referred to above. In fact, whilst in other domestic species the freezing technique has led to a standardization of the preservation procedures, this technique has not yet yielded satisfactory results in pigs.

In addition, in order to enhance breeding productivity in pigs there is an increasingly urgent demand on the part of operators for being able to carry out a single A.I. intervention so as to reduce operating costs.

### Summary of invention

Now we have unexpectedly found that it is possible to produce microcapsules containing active seminal material.

Consequently, the present invention relates to microcapsules comprising:
a) a liquid nucleus containing a suspension of pig seminal material and a biodegradable and/or a biocompatible polymer,
b) a film consisting of a possibly cross-linked alginate of a bivalent or trivalent metal, possibly reticulated.

With this type of preparations it is possible both to overcome the drawbacks linked to the limited stability of the seminal material and to enable the use of a single operation of artificial insemination, with considerable economic advantages resulting from the drastic reduction in the intervention of the operator and from the use of a reduced amount of seminal material. Furthermore, the seminal material is appropriately protected and is released from the formulation that forms the subject of the present patent over a prolonged time range.

It must moreover be emphasized that a further complication of fertilization in pigs is linked to the ovulation times and modalities that are typical of this species. In fact, the fertile period is very short and may vary according to the seasonal period, as was said previously. For this reason, at present a single intervention of fertilization with diluted and refrigerated seminal material does not provide sufficient guarantees of success for a complete fertilization of the egg cells.

We have now found and experimentally proved that, with the micro-encapsulation of pig seminal material, in which no type of reaction within the nucleus containing the seminal material is expected, it is possible to protect pig semen, which is characterized by high instability.

As a result, we obtain the encapsulation of pig semen in the above-mentioned biocompatible and biodegradable polymer, which enables prolonged and controlled release of the active seminal material. In this way, it is possible to maintain high concentrations of spermatozoa in the uterus for the entire duration of oestrus, and consequently bring about an increase in fertility and prolificacy. In addition, the use of micro-encapsulated pig seminal material enables a single operation of artificial insemination to be carried out, in so far as the active spermatozoa are released in a time range that covers the entire duration of oestrus of the sow.

The present invention further relates to the new technique of preparation of the microcapsules according to the present invention, which in particular comprises the following steps:
1. Pig seminal material is suspended in a suitable amount of solution, normally indicated as diluter, generally consisting of an aqueous solution containing: glucose in a concentration of between 0.5 and 10%, sodium bicarbonate in a concentration of between 0.02 and 4.0 %, potassium chloride in a concentration of between 0.01 and 3.0 %, penicillin in a concentration of between 10² U.l/l and 10¹⁰ U.l./l, streptomycin in concentration of between 0.01 and 2.0 %, or another antibiotic normally used for this purpose in suitable concentrations, so as to obtain a concentration of spermatozoa of from 1 x 10⁴ up to a 5 x 10¹⁰ cells/ml,
2. the biocompatible and/or biodegradable polymer and then an aqueous solution of halides of bivalent metals, such as salts of calcium, strontium, zinc, or halides of trivalent metals, such as aluminium, iron, chromium, etc., are added to the suspension obtained in step (1),
3. the suspension resulting from step (2) is added, drop by drop, to a solution of sodium alginate, keeping the temperature of the entire system within a range of between 5°C and 45°C, and microcapsules of a gelatinous type are obtained, which are subsequently dispersed in the diluter for pig semen described previously,
4. the microcapsules obtained in the previous step may possibly be reticulated by means of suspension in an aqueous solution of a cross-linked agent of a polyamine type, which is maintained under stirring, to bring rigid microcapsules.

### Detailed description of invention

The microcapsules according to the present invention may therefore be of a gelatinous or of a rigid type, according to whether the outer film consisting of an alginate of a bivalent or trivalent metal has not undergone or has undergone cross-linking.

The microcapsules of a gelatinous type consist of a liquid nucleus containing the suspension of seminal material and biodegradable polymer, and of a bio-erodible gelatinous film of non-crosslinked alginate.

The biodegradable and/or biocompatible polymeric material according to the present invention contained in the liquid nucleus of the microcapsules that form the subject of the present invention is preferably chosen from among the group consisting of glucans, scleroglucans, mannans, galactomannans, gellans, carrageenans, chitosans, pectins, xanthanes, polyanhydrides, polyamino acids, poly-(methyl vinyl ethers / maleic anhydride), carboxymethyl cellulose and derivatives, sodium carboxymethyl cellulose, polyvinyl alcohols, hydroxypropyl cellulose having a mean molecular weight of from 2,000 to 4,000,000, but preferably of from 10,000 to 2,000,000, hydroxypropyl methylcellulose, carboxyvinyl polymers, alginic acid and derivatives thereof, polyvinyl alcohols, ethylcellulose, methylcellulose and cellulose derivatives in general, starches, starch derivatives, as well as alpha-, beta-, gamma-cyclodextrins and dextrin derivatives in general. For all the polymers referred to there exist different types available on the market characterized by different physical-chemical properties of solubility and gelation.

These polymers are generally present in concentrations of between 1.0 and 90% of the total weight of the microcapsule, but preferably between 20 and 80%.

Preferably, hydroxypropyl methylcellulose is used, having different molecular weights, in general of between 1000 and 4,000,000 Daltons, and preferably of between 10,000 and 1,500,000 and a different degree of methoxyl substitution, in general between 15 and 30%, and a degree of hydroxypropoxyl substitution of between 4 and 30%, as biodegradable and/or biocompatible polymer for the liquid nucleus of the microcapsules according to the present invention. Consequently, this polymer presents a prevalently erodible or gellable nature, according to the viscosity and degree of substitution in the polymeric chain.

According to a particularly preferred solution, hydroxypropyl methylcellulose available on the market under the registered trade-mark METHOCEL® E 50 with a molecular weight of 22,000 and with a degree of methoxyl substitution 29 and hydroxypropoxyl substitution of 8.5 is used.

The alginates of the bivalent metals forming the microcapsules that form the subject of the present invention are generally those of calcium, strontium, and zinc. The alginates of the trivalent metals are, for example, those of aluminium, iron, and chromium.

Preferably, the film of the microcapsules according to the present invention is made up of calcium alginate or zinc alginate.

The alginates forming the film of the microcapsules according to the present invention generally present, at 2% in aqueous solution, a viscosity of between 200 cps and 20,000 cps at a temperature of 25°C.

The capsules obtained are generally characterized by having a dimension of between 50 micron and 8 mm, preferably of between 100 micron and 5.0 mm, and this enables easy manipulation, transportation and preservation, as well as the use of ordinary catheters for artificial insemination.

In step (1) of the process according to the present invention, the diluter for pig seminal material contains glucose in a concentration of preferably between 1.5 and 5 wt%, sodium bicarbonate in a concentration of between 0.05 and 0.8%, potassium chloride in a concentration preferably of between 0.01 and 0.10%, penicillin in a concentration preferably of between 10⁴ U.l./l and 10⁸ U.l./l, streptomycin in a concentration of between 0.04 and 0.90 wt% or another antibiotic in appropriate concentrations, with the purpose of obtaining a concentration of between 2 x 10⁵ and 5 x 10⁸ cells/ml.

In step (2) of the process that forms the subject of the present invention, the halides of bivalent metals preferably used are calcium chloride or zinc chloride, used in an aqueous solution in a concentration ranging from 0.001 M to 2.5 M, but preferably from 0.01 to 1.0 M.

Step (3) is preferably conducted at a temperature of between 10 and 25°C, and the suspension is extruded through hypodermic needles of appropriate size and aperture by means of a peristaltic pump, at a rate such as to produce approximately 20 drops/min. As the metal ions diffuse towards the outer surface, in particular of the drop, they react with the alginate to form a film of alginate of an alkaline earth metal (e.g., calcium alginate), which enables sufficiently rigid microcapsules to be obtained. These are washed a number of times, preferably with a physiological solution, or preferably with the diluter. These microcapsules of a "gelatinous type" may constitute of themselves, without undergoing any further treatment, a first type of material usable for A.I.

In step (4), the capsules of a "gelatinous type" prepared as described may undergo cross-linking by means of interface polymerization of the alginate. This procedure brings about the conversion of the gelatinous membrane into a semipermeable rigid membrane of alginate cross-linking with a cross-linked agent of the polyamine type. Preferably these cross-linking agents are selected from: protamine sulphate or phosphate, preferably in the form of an aqueous solution in concentrations of between 0.1 and 5.0% w/v, even more preferably protamine sulphate in a concentration of between 0.5 and 3.0% w/v, polylysine bromohydrate of molecular weight of between 1000 and 800,000 in an aqueous solution at a concentration preferably of between 0.01 and 5.0% w/v, even more preferably of between 0.03 and 3.0% w/v, and polyvinyl amine in a concentration of from 0.001 to 5.0% w/v, even more preferably of from 0.01 to 1.0% w/v.

Step (4) is moreover preferably carried out according to the following operating procedure:
stirring rate: 5-300 rpm
reaction times: 2-180 min.

The capsules that are obtained and that may be defined of the "rigid type" are filtered and respectively washed and suspended in the same diluter for pig semen, previously described.

The following examples are given to provide illustrations of the preparation of the capsules that form the subject of the present invention.

### EXAMPLE 1

### Preparation of the capsules (GELATINOUS TYPE)

Hydroxypropyl methylcellulose (Methocel E50 Premium EP) (4 % w/v) and calcium chloride (0.3 M) is added to a suspension of seminal material of Large White boar with a concentration of 26 x 10⁶ cells/ml (motility 90%, acrosomal integrity of over 90%), and the resulting suspension, to which is added the polymeric material, is in turn added to an aqueous solution of sodium alginate (0.5 % w/v), and kept under gentle stirring (30 rpm). The drops are produced by extrusion through hypodermic needles (25G x 5/8") by means of a peristaltic pump (Esapump, Advanced Products, Milan, Italy), at a rate such as to produce approximately 20 drops/min.

As the calcium ions diffuse towards the outer surface of the drop, they react with the alginate to form a film of calcium alginate. The capsules thus obtained are filtered, washed three times, and suspended in a physiological solution, and suspended in diluter for pig semen consisting of an aqueous solution containing 2.9% glucose, 0.2% sodium bicarbonate, 0.03% of potassium chloride, 10⁶ U.l./l of penicillin, and 0.1% streptomycin.

The capsules thus consist of a liquid nucleus containing the suspension of seminal material and hydroxypropyl methylcellulose, and a bio-erodible gelatinous membrane. These capsules (GELATINOUS TYPE) may constitute a first type of material usable for A.I..

Of the preparation obtained in Example 1 Table I gives the results of the determinations of survival time of the seminal material, acrosomal integrity and motility; these latter two parameters are expressed in percentages.

### EXAMPLE 2

### Preparation of the capsules (RIGID TYPE)

The GELATINOUS TYPE capsules, prepared as described in Example 1, starting from a suspension of seminal material (26 x 10⁶ cells/ml) are suspended in an aqueous solution of protamine sulphate (1% w/v), kept under stirring (30 rpm) for 20 min. By means of this procedure the interface polymerization of the calcium alginate is obtained, and capsules of the RIGID TYPE are prepared. This procedure determines the conversion of the gelatinous membrane into a semipermeable rigid membrane of alginate cross-linked with protamine. The capsules are filtered, washed three times with physiological solution and suspended in diluter for pig semen.

The results of the controls carried out are given in Table I.

### Example 3

### Preparation of capsules (RIGID TYPE)

The procedure described in Example 2 is used, with the only difference that a suspension of seminal material with a concentration of 52 x 10⁶ cells/ml is used.

The results of the controls carried out are given in Table I.

**Table 1**

| Code | Survival time (h) | Acrosomal integrity (%) | Motility (%) |
|---|---|---|---|
| Fresh spermatic material | 96.0 | 90.0 | 60.0 |
| Example 1 | 72.0 | 35.0 | 5.0 |
| Example 2 | 96.0 | 68.0 | 20.0 |
| Example 3 | 48.0 | 40.0 | 10.0 |

From the results reported in Table I, it is evident that the preparation described in Example 2 presents a survival time of the seminal material that is identical to that of the fresh spermatic material, an acrosomal integrity of 75%, as compared to the fresh seminal material, and a good motility as compared to the other preparations.

## Claims

1. Microcapsules comprising:
a) a liquid nucleus containing a suspension of pig seminal material and a biodegradable and/or biocompatible polymer,
b) a film consisting of a possibly cross-linked alginate of a bivalent or trivalent metal.

2. Microcapsules according to claim 1, **characterized in that** they are gelatinous and the film of alginate is not cross-linked and is bio-erodible.

3. The microcapsules according to claim 1 of a rigid type, **characterized in that** the film of alginate is cross-linked.

4. The microcapsules according to any one of claims 1-3, **characterized in that** the biodegradable and/or biocompatible polymer is chosen from the group consisting of: glucans, scleroglucans, mannans, galactomannans, gellans, carrageenans, chitosans, pectins, xanthanes, polyanhydrides, polyamino acids, poly-(methyl vinyl ethers / maleic anhydride), carboxymethyl cellulose and derivatives thereof, sodium carboxymethyl cellulose, polyvinyl alcohols, hydroxypropyl cellulose with a mean molecular weight of from 2,000 to 4,000,000, hydroxypropyl methylcellulose, carboxyvinyl polymers, alginic acid and derivatives thereof, polyvinyl alcohols, ethylcellulose, methylcellulose and cellulose derivatives in general, starches, derivatives of starches, alpha- beta- gamma-cyclodextrins and derivatives of dextrins.

5. The microcapsules according to any one of claims 1-5, **characterized in that** said biodegradable polymer is chosen from the group consisting of hydroxypropyl cellulose with a mean molecular weight of between 10,000 and 2,000,000.

6. The microcapsules according to any one of claims 1-4, **characterized in that** hydroxypropyl methylcellulose with molecular weight between 10,000 and 1,500,000, and degree of methoxyl substitution of between 15 and 30%, and degree of substitution with hydroxypropoxyl of between 4 and 30% is used as biodegradable and/or biocompatible polymer.

7. The microcapsules according to any one of claims 1-6, **characterized in that** said biodegradable and/or biocompatible polymers are contained in concentrations of between 1.0 and 90% of the total weight of the microcapsule.

8. The compositions according to any one of claims 1-7, **characterized in that** the said biodegradable polymers are contained in concentrations of between 20 and 80% of the total weight of the microcapsule.

9. The microcapsules according to any one of claims 1-8, **characterized in that** the film of alginate of a bivalent metal is selected from alginate of calcium, strontium, and zinc; the alginates of the trivalent metals are selected from those of aluminium, iron, and chromium.

10. The microcapsules according to any one of claims 1-9, **characterized in that** the film consists of alginate of calcium or of zinc.

11. The microcapsules according to any one of claims 1-10, **characterized in that** they present, in 2% aqueous solutions, a viscosity of between 200 cps and 20,000 cps at a temperature of 25°C.

12. The capsules according to any one of claims 1-11, **characterized in that** they have dimensions of between 50 micron and 8 mm.

13. The microcapsules according to any one of claims 1-12, **characterized in that** they have dimensions of between 100 micron and 5.0 mm.

14. Process for preparing the microcapsules according to any one of claims 1-13 comprising the following steps:
1. Pig seminal material is suspended in a suitable amount of solution, consisting of an aqueous solution containing: glucose in a concentration of between 0.5 and 10%, sodium bicarbonate in a concentration of between 0.02 and 4.0 %, potassium chloride in a concentration of between 0.01 and 3.0 %, penicillin in a concentration of between 10² U.l/l and 10¹⁰ U.l./L, streptomycin in concentration of between 0.01 and 2.0 %, or other antibiotics normally used for this purpose in suitable concentrations, so as to obtain a concentration of spermatozoa of from 1 x 10⁴ up to 5 x 10¹⁰ cells/ml;
2. the biocompatible and/or biodegradable polymer and then an aqueous solution of halides of bivalent metals or halides of trivalent metals is added to the suspension obtained in step (1),
3. the suspension resulting from step (2) is added, drop by drop, to a solution of sodium alginate, keeping the temperature of the entire system within a range of between 5°C and 45°C, and microcapsules of a gelatinous type are obtained, which are subsequently dispersed in the diluter for pig semen described previously;
4. the microcapsules obtained in the foregoing step may possibly be reticulated by means of suspension in an aqueous solution of a cross-linking agent of a polyamine type, which is kept under stirring, to give rise to rigid microcapsules.

15. Process according to claim 14, **characterized in that** in step (1) the diluter for pig seminal material contains glucose in a concentration of between 1.5 and 5 wt%, sodium bicarbonate in a concentration of between 0.05 and 0.8%, potassium chloride in a concentration preferably of between 0.01 and 0.10%, penicillin in a concentration preferably of between 10⁴ U.l./l and 10⁸ U.l./l, streptomycin in a concentration of between 0.04 and 0.90 wt%, with the purpose of obtaining a concentration of between 2 x 10⁵ and 5 x 10⁸ cells/ml.

16. Process according to either one of the claims 14 and 15, **characterized in that** in step (2), halides of bivalent metals are calcium chloride or zinc chloride, used in an aqueous solution in a concentration ranging from 0.001 M to 2.5 M.

17. Process according to claim 16, **characterized in that** the concentration of calcium chloride and zinc chloride is between 0.01 and 1.0 M.

18. Process according to any one of the claims 14-17, **characterized in that** step (3) is conducted at a temperature of between 10 and 25°C, and the suspension is extruded through hypodermic needles of appropriate size and aperture.

19. Process according to any one of the claims 14-18, **characterized in that** in step (4) the cross-linking agent is selected from: protamine sulphate or phosphate, preferably in the form of an aqueous solution in concentrations of between 0.1 and 5.0% w/v, polylysine bromohydrate of molecular weight of between 1000 and 800,000 in an aqueous solution at a concentration of between 0.001 and 5.0% w/v, and polyvinyl amine at a concentration of between 0.001 and 5.0% w/v.

20. Process according to any one of the claims 14-19, **characterized in that** in step (4) the cross-linking agent is selected from: protamine sulphate or phosphate, in the form of an aqueous solution in concentrations of between 0.5 and 3.0% w/v, bromohydrate poly l-lysine of molecular weight of between 1000 and 800,000 in an aqueous solution at a concentration of between 0.03 and 3.0% w/v, and polyvinyl amine in a concentration of from 0.01 to 1.0% w/v.

21. Process according to any one of the claims 14-20, **characterized in that** it is carried out for a time ranging between 2 min and 180 min.

22. The microcapsules according to any one of the claims 1-12 for artificial insemination of pigs.

## Patentansprüche

1. Mikrokapseln, die wie folgt enthalten:
a) einen flüssigen Kern mit einer Suspension aus Schweinesperma und einem biologisch abbaubaren und/oder biokompatiblen Polymer,
b) einen Film, der sich aus möglicherweise quervernetztem Alginat eines bivalenten oder trivalenten Metalls zusammensetzt.

2. Mikrokapseln gemäß Anspruch 1, die dadurch charakterisiert sind, dass sie aus Gelatine bestehen, der Alginatfilm keine Quervernetzungen aufweist und biologisch abbaubar ist..

3. Die Mikrokapseln gemäß Anspruch 1 vom festen Typ, die dadurch charakterisiert sind, dass der Alginatfilm quervernetzt ist.

4. Die Mikrokapseln gemäß einem der Ansprüche von 1-3, die dadurch charakterisiert sind, dass das biologisch abbaubare und/oder biokompatible Polymer aus der Stoffgruppe ausgewählt wird, die Glukane, Skleroglukane, Mannane, Galaktomannane, Gellane, Carrageene, Chitosane, Pektine, Xanthane, Polyanhydride, Polyaminosäuren, Poly-(methylvinyläther / maleinsäureanhydrid), Carboxymethylcellulose und Derivate davon, Natriumcarboxymethylcellulose, Polyvinylalkohole, Hydroxypropylcelluose mit einem mittleren Molekulargewicht von 2.000 bis 4.000.000, Hydroxypropylmethylcellulose, Carboxyvinylpolymere, Alginsäure und Derivate davon, Polyvinylalkohole, Ethylcellulose, Methylcellulose und Cellulosederivate im Allgemeinen, Stärken, Stärkederivate, alpha-, beta- und gamma-Cyclodextrine und Dextrinderivate beinhaltet.

5. Die Mikrokapseln gemäß einem der Ansprüche von 1-5, die dadurch charakterisiert sind, dass das besagte biologisch abbaubare Polymer aus der Stoffgruppe stammt, die Hydroxylpropylcellulose mit einem mittleren Molekulargewicht von 10.000 bis 2.000.000 enthält.

6. Die Mikrokapseln gemäß einem der Ansprüche von 1-4, die dadurch charakterisiert sind, dass Hydroxypropylmethylcelluose mit einem Molekulargewicht zwischen 10.000 und 1.500.000 und einem Methoxylsubstitutionsgrad zwischen 15 und 30% und einem Hydroxypropoxylsubstitutionsgrad zwischen 4 und 30% als biologisch abbaubares und/oder biokompatibles Polymer eingesetzt wird.

7. Die Mikrokapseln gemäß einem der Ansprüche von 1-6, die dadurch charakterisiert sind, dass die besagten biologisch abbaubaren und/oder biokompatiblen Polymere in Konzentrationen mit einem Anteil von 1,0 bis 90 % am Gesamtgewicht der einzelnen Mikrokapsel beteiligt sind.

8. Die Zusammensetzungen gemäß einem der Ansprüche von 1-7, die dadurch charakterisiert sind, dass die besagten biologisch abbaubaren Polymere in Konzentrationen von 20 bis 80 % des Gesamtgewichtes der einzelnen Mikrokapseln enthalten sind.

9. Die Mikrokapseln gemäß einem der Ansprüche von 1-8, die dadurch charakterisiert sind, dass der Alginatfilm mit einem bivalenten Metall aus Kalzium-, Strontium- und Zinkalginat ausgewählt wird; die Alginate trivalenter Metalle werden ausgewählt unter Aluminium-, Eisen und Chromalginat.

10. Die Mikrokapseln gemäß einem der Ansprüche von 1-9, die dadurch charakterisiert sind, dass der Film aus Kalzium- oder Zinkalginat besteht.

11. Die Mikrokapseln gemäß einem der Ansprüche von 1-10, die dadurch charakterisiert sind, dass sie in einer 2%igen wässrigen Lösung bei einer Temperatur von 25° C eine Viskosität von 200 cps bis 20.000 cps aufweisen.

12. Die Kapseln gemäß einem der Ansprüche von 1-11, die dadurch charakterisiert sind, dass sie Größenordnungen von 50 Mikrometer bis 8 mm erreichen.

13. Die Mikrokapseln gemäß einem der Ansprüche von 1-12, die dadurch charakterisiert sind, dass sie eine Größenordnung von 100 Mikrometer bis 5,0 mm erreichen.

14. Der Prozess zur Herstellung der Mikrokapseln gemäß einem der Ansprüche von 1-13 umfasst die folgenden Schritte:
1. Spermienmaterial von Schweinen wird in einer entsprechenden Menge einer Lösung aufgenommen, die sich zusammensetzt aus 0,5 bis 10% Glukose, 0,02 bis 4 % Natriumbikarbonat, 0,01 bis 3,0 % Kaliumchlorid, Penicillin in einer Konzentration von 10² bis 10¹⁰ I.E/1, Streptomycin in einer Konzentration von 0,01 bis 2,0 % oder andere Antibiotika in angemessenen Konzentrationen, die zu diesem Zweck normalerweise eingesetzt werden, um so eine Spermienkonzentration von 1 x 10⁴ bis zu 5 x 10¹⁰ Zellen/ml zu erhalten;
2. anschließend wird zuerst das biokompatible und/oder biologisch abbaubare Polymer und dann eine wässrige Lösung von Halogeniden bivalenter Metalle oder Halogeniden trivalenter Metalle zu der in Schritt (1) erhaltenen Suspension zugegeben.
3. die in Schritt (2) entstandene Suspension wird tropfenweise zu einer Lösung aus Natriumalginat zugegeben, wobei die Temperatur des gesamten Systems innerhalb eines Bereiches zwischen 5° C und 45° C gehalten wird und die Mikrokapseln aus Gelatine entstehen, die nachfolgend im Verdünner für Schweinespermien, wie zuvor beschrieben, dispergiert werden;
4. die im vorherigen Schritt entstandenen Mikrokapseln können durch die Suspension in einer wässrigen Lösung eines quervernetzenden Agens vom Polyamintyp unter ständigem Rühren eine netzförmige Struktur erhalten, um so feste Mikrokapseln entstehen zu lassen.

15. Der Prozess entsprechend dem Anspruch 14, der dadurch charakterisiert ist, dass der in Schritt (1) verwendete Verdünner für das Schweinespermienmaterial Glukose in einer Konzentration von 1,5 bis 5 Gewicht%, 0,05 bis 0,8% Natriumbicarbonat, Kaliumchlorid vorzugsweise in einer Konzentration von 0,01 bis 0,10%, vorzugsweise 10⁴ I.E/l bis 10⁶ I.E/l Penicillin, Streptomycin in einer Konzentration von 0,04 bis 0,90 Gewicht% enthält in der Absicht, eine Konzentration von 2 x 10⁸ bis 5 x 10⁸ Zellen/ml zu erhalten.

16. Der Prozess gemäß einem der Ansprüche 14 und 15, der dadurch charakterisiert ist, dass es sich bei den in Schritt (2) enthaltenen Halogeniden bivalenter Metalle um Kalzium- oder Zinkchlorid handelt, die in einer wässrigen Lösung in einer Konzentration von 0,001M bis 2,5M vorliegen.

17. Der Prozess gemäß dem Anspruch 16, der dadurch charakterisiert ist, dass die Konzentration von Kalzium- und Zinkchlorid zwischen 0,01 und 0,1M liegt.

18. Der Prozess gemäß einem der Ansprüche von 14-17, der dadurch charakterisiert ist, dass der Schritt (3) bei einer Temperatur im Bereich von 10 bis 25° C durchgeführt wird und die Suspension durch Injektionsnadeln entsprechender Größe und Öffnung abgezogen wird.

19. Der Prozess gemäß einem der Ansprüche von 14-18, der dadurch charakterisiert ist, dass das in Schritt (4) quervernetzende Agens ausgewählt wird unter: Protaminsulfat oder -phosphat, vorzugsweise in Form einer wässrigen Lösung in Konzentrationen von 0,1 bis 5,0% w/v, Polylysinbromhydrat mit einem Molekulargewicht von 1.000 bis 800.000 in einer wässrigen Lösung mit einer Konzentration von 0,001 bis 5,0% w/v und Polyvinylamin in einer Konzentration von 0,001 bis 5% w/v.

20. Der Prozess gemäß einem der Ansprüche von 14-19, der dadurch charakterisiert ist, dass das in Schritt (4) quervernetzende Agens ausgewählt wird unter: Protaminsulfat oder -phosphat in Form einer wässrigen Lösung in Konzentrationen von 0,5 bis 3,0% w/v, Bromhydrat-poly-L-Lysin mit einem Molekulargewicht zwischen 1.000 und 800.000 in einer wässrigen Lösung mit einer Konzentration zwischen 0,03 und 3,0% w/v und Polyvinylamin in einer Konzentration zwischen 0,01 und 1,0% w/v.

21. Der Prozess gemäß einem der Ansprüche von 14-20, der dadurch charakterisiert ist, dass er in einem Zeitrahmen von 2 min. bis 180 min. durchgeführt wird.

22. Die Mikrokapseln gemäß einem der Ansprüche von 1-12 zur künstlichen Insemination von Schweinen.

## Revendications

1. Microcapsules comprenant :
a) un noyau liquide contenant une suspension de matériel séminal de porc et un polymère biodégradable et/ou biocompatible,
b) un film consistant en un alginate d'un métal bi ou trivalent éventuellement réticulé.

2. Microcapsules selon la revendication 1, **caractérisées en ce qu'**elles sont gélatineuses et que le film d'alginate n'est pas réticulé et est bio-érodable.

3. Microcapsules selon la revendication 1 de type rigide, **caractérisées en ce que** le film d'alginate est réticulé.

4. Microcapsules selon l'une quelconque des revendications 1 à 3, **caractérisées en ce que** le polymère biodégradable et/ou biocompatible est choisi dans le groupe constitué de : glycanes, scléroglycanes, mannanes, galactomannanes, gellanes, carraghénanes, chitosanes , pectines, xanthanes, polyanhydrides, polyacides aminés, poly-(méthyl vinyl éthers /anhydride maléique), carboxyméthyl cellulose et ses dérivés, carboxyméthyl cellulose de sodium, alcools polyvinyliques, hydroxypropyl cellulose avec une masse moléculaire moyenne de 2 000 à 4 000 000, hydroxypropyl méthylcellulose, polymères carboxyvinyliques, acide alginique et ses dérivés, alcools polyvinyliques, éthylcellulose, méthylcellulose et dérivés de cellulose en général, amidons, dérivés d'amidons, alpha- bêta-gamma-cyclodextrines et dérivés des dextrines.

5. Microcapsules selon l'une quelconque des revendications 1 à 5, **caractérisées en ce que** ledit polymère biodégradable est choisi dans le groupe consistant en l'hydroxypropyl cellulose avec une masse moléculaire moyenne comprise entre 10 000 et 2 000 000.

6. Microcapsules selon l'une quelconque des revendications 1 à 4, **caractérisées en ce que** l'hydroxypropyl méthylcellulose avec une masse moléculaire entre 10 000 et 1 500 000, et un degré de substitution du méthoxyle compris entre 15 et 30%, et un degré de substitution avec l'hydroxypropoxyle compris entre 4 et 30% est utilisé en tant que polymère biodégradable et/ou biocompatible.

7. Microcapsules selon l'une quelconque des revendications 1 à 6, **caractérisées en ce que** lesdits polymères biodégradables et/ou biocompatibles sont contenus en concentrations comprises entre 1,0 et 90% du poids total de la microcapsule.

8. Compositions selon l'une quelconque des revendications 1 à 7, **caractérisées en ce que** lesdits polymères biodégradables sont contenus en concentrations comprises entre 20 et 80% du poids total de la microcapsule.

9. Microcapsules selon l'une quelconque des revendications 1 à 8, **caractérisées en ce que** le film d'alginate d'un métal bivalent est sélectionné parmi l'alginate de calcium, de strontium, et de zinc ; les alginates des métaux trivalents sont sélectionnés parmi ceux d'aluminium, de fer, et de chrome.

10. Microcapsules selon l'une quelconque des revendications 1 à 9, **caractérisées en ce que** le film est constitué d'alginate de calcium ou de zinc.

11. Microcapsules selon l'une quelconque des revendications 1 à 10, **caractérisées en ce qu'**elles présentent, en solutions aqueuses à 2%, une viscosité entre 200 cps et 20 000 cps à une température de 25°C.

12. Capsules selon l'une quelconque des revendications 1 à 11, **caractérisées en ce qu'**elles ont des dimensions comprises entre 50 microns et 8 mm.

13. Microcapsules selon l'une quelconque des revendications 1 à 12, **caractérisées en ce qu'**elles ont des dimensions comprises entre 100 microns et 5,0 mm.

14. Procédé pour préparer les microcapsules selon l'une quelconque des revendications 1 à 13 comprenant les étapes suivantes :
1. le matériel séminal de porc est mis en suspension dans une quantité de solution appropriée, constituée d'une solution aqueuse contenant : du glucose en une concentration comprise entre 0,5 et 10%, du bicarbonate de sodium en une concentration comprise entre 0,02 et 4,0%, du chlorure de potassium en une concentration comprise entre 0,01 et 3,0%, de la pénicilline en concentration comprise entre 10² U.l/l et 10¹⁰ U.l/l, de la streptomycine en concentration comprise entre 0,01 et 2,0%, ou d'autres antibiotiques normalement utilisés dans ce but en concentrations appropriées, pour obtenir une concentration de spermatozoïdes de 1×10⁴ jusqu'à 5×10¹⁰ cellules/ml ;
2. le polymère biocompatible et/ou biodégradable et ensuite une solution aqueuse d'halides de métaux bivalents ou d'halides de métaux trivalents est ajoutée à la suspension obtenue à l'étape (1),
3. la suspension résultant de l'étape (2) est ajoutée, goutte à goutte, à une solution d'alginate de sodium, en maintenant la température de l'ensemble du système dans une plage comprise entre 5°C et 45°C, et des microcapsules d'un type gélatineux sont obtenues, qui sont subséquemment dispersées dans le diluant de la semence de porc décrit plus haut ;
4. les microcapsules obtenues à l'étape précédente peuvent éventuellement être réticulées à l'aide d'une suspension dans une solution aqueuse d'un agent de réticulation de type polyamine, qui est maintenue sous agitation, pour donner lieu à des microcapsules rigides.

15. Procédé selon la revendication 14, **caractérisé en ce que** dans l'étape (1) le diluant pour le matériel séminal du porc contient du glucose en concentration comprise entre 1,5 et 5% en poids, du bicarbonate de sodium en une concentration comprise entre 0,05 et 0,8%, du chlorure de potassium en une concentration de préférence comprise entre 0,01 et 0,10%, de la pénicilline en concentration de préférence comprise entre 10⁴ U.l/l et. 10⁸ U.l/l, de la streptomycine en concentration comprise entre 0,04 et 0.90% en poids, avec le but d'obtenir une concentration comprise entre 2×10⁵ et 5×10⁸ cellules/ml.

16. Procédé selon l'une quelconque des revendications 14 ou 15, **caractérisé en ce qu'**à l'étape (2), les halides de métaux bivalents sont le chlorure de calcium ou le chlorure de zinc, utilisés en solution aqueuse à une concentration allant de 0,001 à 2,5 M.

17. Procédé selon la revendication 16, **caractérisé en ce que** la concentration en chlorure de calcium et chlorure de zinc est comprise entre 0,01 et 1,0 M.

18. Procédé selon l'une quelconque des revendications 14 à 17, **caractérisé en ce que** l'étape (3) est menée à une température comprise entre 10 et 25°C, et la suspension est extrudée à travers des aiguilles hypodermiques de taille et ouverture appropriées.

19. Procédé selon l'une quelconque des revendications 14 à 18, **caractérisé en ce qu'**à l'étape (4) l'agent de réticulation est sélectionné parmi : le sulfate ou le phosphate de protamine, de préférence sous forme d'une solution aqueuse en concentrations comprises entre 0,1 et 5,0% p/v, du bromohydrate de polylysine de masse moléculaire comprise entre 1 000 et 800 000 en solution aqueuse à une concentration comprise entre 0,001 et 5,0% p/v, et de l'amine polyvinylique à une concentration comprise entre 0,001 et 5,0% p/v.

20. Procédé selon l'une quelconque des revendications 14 à 19, **caractérisé en ce qu'**à l'étape (4) l'agent de réticulation est sélectionné parmi : le sulfate ou le phosphate de protamine, sous forme d'une solution aqueuse de concentrations comprises entre 0,5 et 3,0% p/v, de la poly l-lysine de bromohydrate de masse moléculaire comprise entre 1 000 et 800 000 en solution aqueuse à une concentration comprise entre 0,03 et 3,0% p/v, et de l'amine polyvinylique en une concentration allant de 0,01 à 1,0% p/v.

21. Procédé selon l'une quelconque des revendications 14 à 20, **caractérisé en ce qu'**il est exécuté pour un intervalle de temps compris entre 2 min et 180 min.

22. Microcapsules selon l'une quelconque des revendications 1 à 12 pour l'insémination artificielle de porcs.
